# FASCICULE DE BREVET EUROPEEN

(11) **EP 3 082 791 B1**
(45) Date de publication et mention de la délivrance du brevet: **20.03.2024**
(21) Numéro de dépôt: 14827204.0
(22) Date de dépôt: 18.12.2014
(51) Int. Cl.: A61K 9/00, A61K 9/20, A61K 31/197, A61P 25/32

(54) **COMPOSITIONS PHARMACEUTIQUES ORALES À RÉTENTION GASTRIQUE À BASE DE BACLOFÈNE**
ORALE PHARMAZEUTISCHE ZUSAMMENSETZUNGEN MIT RETENTION IM MAGEN ENTHALTEND BACLOFEN
GASTRO-RETENTIVE ORAL PHARMACEUTICAL COMPOSITIONS COMPRISING BACLOFEN

(30) Priorité: 18.12.2013 FR 1362916
(43) Date de publication de la demande: 26.10.2016
(73) Titulaire: Ethypharm, 92210 Saint-Cloud (FR)
(72) Inventeur: HERRY, Catherine, F-27670 Saint-Ouen du Tilleul (FR); CONTAMIN, Pauline, F-76220 La Feuillie (FR)
(74) Mandataire: Regimbeau
(86) Numéro de dépôt international: PCT/EP2014/078597
(87) Numéro de publication internationale: WO 2015/091874

(56) Documents cités:
- EP-A1- 1 745 775
- EP-A2- 1 849 462
- WO-A1-03/011255
- WO-A1-2010/064100
- WO-A1-2011/151708
- WO-A2-2011/101866
- S Gande ET AL: "Sustained-release effervescent floating matrix tablets of baclofen: development, optimization and in vitro-in vivo evaluation in healthy human volunteers", Daru : journal of Faculty of Pharmacy, Tehran University of Medical Sciences, 2011, pages 202-209, XP055120611, Iran Extrait de l'Internet: URL:http://www.pubmedcentral.nih.gov/artic lerender.fcgi?artid=3232112&tool=pmcentrez &rendertype=abstract [extrait le 2014-05-28]

## Description

L'invention concerne une formulation galénique orale de molécule BCS de classe III comme le baclofène sous forme de comprimé à libération contrôlée par rétention gastrique. Ces compositions peuvent être administrées une fois ou deux fois par jour.

La voie orale est la voie envisagée en premier lieu lors de la mise en forme d'une nouvelle entité pharmaceutique de par l'observance au traitement qu'elle induit. Cependant, cette voie est abandonnée pour de nombreuses molécules en développement en raison de leur faible biodisponibilité orale. Celle-ci peut être due à divers facteurs provenant des propriétés même de la molécule ou bien de la physiologie du tractus gastro-intestinal *(Fasinu, et al., 2011).* Diverses approches ont été explorées ces dix dernières années pour améliorer la biodisponibilité orale de ces molécules incluant des moyens physiques ou chimiques.

La classification de molécules BCS (Biopharmaceutics Classification System) est un outil significatif utilisé pour le développement des formes orales dans l'industrie pharmaceutique et est notamment adopté par la FDA, l'EMEA et l'OMS *(Dahan, et al., 2009).* Celle-ci se divise en quatre catégories de molécules et se base sur les éléments fondamentaux qui contrôlent l'absorption orale que sont la perméabilité membranaire intestinale ainsi que la solubilité d'une molécule dans le milieu gastro-intestinal. Une molécule est considérée soluble si la dose maximale d'une forme à libération immédiate est soluble dans 250 ml ou moins d'un milieu aqueux dont le pH est compris entre 1,2 et 6,8 et est considérée perméable si son absorption à travers la membrane intestinale est supérieure ou égale à 90 %. Une molécule BCS appartenant à la classe III est hautement soluble et faiblement perméable. Cette dernière particularité est le facteur limitant la biodisponibilité orale et peut mener à l'abandon du développement de formulation orale de molécules ayant pourtant un fort potentiel thérapeutique.

Le baclofène (ci-dessous) est une molécule BCS appartenant à la classe III. Ses caractéristiques physico-chimiques sont résumées dans le tableau 1.

**Tableau 1 : Caractéristiques physico-chimiques du baclofène (rapport interne)**

| 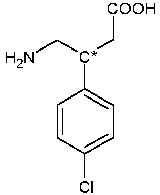 | |
|---|---|
| Masse molaire | 213.66 g/mol |
| Point de fusion | 180-191°C |
| pH d'une solution saturée | 6,5 |
| pKa | pKa₁ = 3.87 |
| | pKa₂ = 9.62 |
| Solubilités | • Soluble : HCl 0.1N, NaOH 0.1N |
| | • Légèrement soluble : eau (3 mg/ml) |
| | • Très légèrement soluble : Ethanol, Méthanol |
| Log P | -1 |

Aux doses usuelles thérapeutiques, le baclofène est connu pour avoir une bonne biodisponibilité orale. Pourtant, son faible log P indique une certaine hydrophilie et donc une faible perméabilité. Cette observation s'explique par la présence de transporteurs spécifiques au niveau de l'intestin grêle permettant le passage de la molécule. Celui-ci est d'autant plus important au niveau du jéjunum. Aussi, un faible passage au niveau du colon suppose la présence d'un autre mécanisme de passage à travers la barrière intestinale *(Merino, et al., 1989).* Cette zone du système digestif ne contient pas de transporteurs mais la molécule étant hydrophile et de petite taille, un faible passage passif à travers les jonctions serrées est possible. Ainsi, il apparaît que lors d'un besoin en concentration plasmatique plus importante du baclofène, la prise d'une dose plus élevée n'est pas efficace en raison de la saturation des transporteurs. La solution utilisée en clinique est alors la prise de faibles doses à intervalles de temps rapprochés ce qui peut rapidement devenir contraignant pour le patient.

Une augmentation de la lipophilie du baclofène et par là-même de sa perméabilité transcellulaire a été observée grâce à la réalisation de prodrogues d'ester de baclofène *(Leisen, et al., 2003).* De par ces propriétés, une plus grande concentration de prodrogue est retrouvée au niveau du tissu cible, le cerveau, grâce à un passage plus aisé de la barrière hémato-encéphalique. Cependant, il est à noter une affinité plus importante pour la pompe d'efflux P-gp ainsi qu'une hydrolyse partielle de la prodrogue en baclofène ce qui conduit finalement à un taux au niveau du site d'action plus bas en baclofène après une administration de la prodrogue par comparaison avec une administration de baclofène seul.

Une fenêtre d'absorption du baclofène est mise en évidence au niveau de l'intestin grêle en raison de la présence de transporteurs *(Merino, et al., 1989).* Afin d'augmenter la biodisponibilité, cette fenêtre d'absorption pourrait ainsi être mise à profit par des techniques galéniques permettant la rétention de la forme orale au niveau de la fenêtre ou en amont *(Davis, 2005).* En effet, un temps de résidence plus long au niveau du site d'absorption doit permettre en théorie un passage plus important de molécules à travers la barrière intestinale si celles-ci ne sont pas susceptibles de subir de dégradation présystémique avant absorption. Ainsi, des formes bioadhésives au mucus intestinal grâce à l'utilisation de polymères cationiques tels que le chitosane ou bien des formes gastrorétentives permettant le gonflement et la flottaison de la forme au niveau de l'estomac ont fait leur apparition ces dernières années en développement pharmaceutique.

Les formes pharmaceutiques à libération prolongée classiques conviennent donc difficilement.

Le principe des formes gastrorétentives consiste à piéger la formulation dans l'estomac pendant une période prolongée et à libérer lentement le principe actif dans l'estomac, soit immédiatement en amont de la fenêtre d'absorption, ce qui permet une absorption prolongée de la substance active.

Pour ce faire, les formes gastrorétentives doivent flotter dans le bol alimentaire contenu dans l'estomac pour ne pas subir la vidange gastrique et gonfler suffisamment pour ne pas passer le pylore.

Généralement, le temps de vidange gastrique en état de jeûne se produit dans un intervalle de temps de 0 à 2 heures et après avoir mangé dans un intervalle de temps de 4 à 6 heures.

Des formulations gastrorétentives sont connues de l'art antérieur.

US 6,797,283 divulgue l'utilisation de comprimés multicouches constitués d'une couche à haut pouvoir gonflant, et d'une couche contenant le principe actif. Le comprimé est ainsi maintenu dans l'estomac pendant une période prolongée.

US 6, 635,280 divulgue des comprimés suffisamment petits pour être ingérés qui gonflent après l'ingestion.

EP2262484 décrit des compositions pharmaceutiques sous forme de comprimés gastrorétentifs à libération prolongée de deux principes actifs, un opioïde associé à de l'acétaminophène. Ces comprimés sont monolithiques à libération lente et se dégradent par érosion ou combinent une partie à libération rapide et une autre partie à libération lente sous forme d'un comprimé bi-couche. L'action rétentive est assurée par une matrice polymère constituée d'au moins un polymère hydrophile qui gonfle quand il est imbibé de fluide jusqu'à une taille suffisante pour une rétention gastrique. Cependant, ces comprimés peuvent être évacués par vidange gastrique avant même d'avoir gonflé suffisamment pour ne pas passer le pylore. Pour cette raison, ils doivent être administrés conjointement à un repas.

EP 1745775 décrit des compositions pharmaceutiques sous forme de comprimés gastrorétentifs comprenant une substance active granulée avec un mélange d'un agent gélifiant faible, un agent gélifiant fort et un agent générateur de gaz. Cependant, il a été observé que ces comprimés peuvent aussi être évacués par vidange gastrique car le temps à partir duquel les comprimés flottent est trop long.

S. Grande et al : « Sustained-release effervescence floating matrix tablets of baclofen : development, optimization and in vitro-in vivo évaluation in healthy human volunteers" décrit un comprimé matriciel à libération contrôlée de baclofène par rétention gastrique fabriqué par compression directe de granules de baclofène, cellulose microcristalline, méthylcellulose, hydroxypropylméthylcellulose, polyvinylpyrrolidone et bicarbonate de sodium.

L'invention a pour objet de fournir des comprimés matriciels à libération contrôlée de Baclofène qui restent dans l'estomac pendant au moins 2h, de préférence 4 heures, parce qu'ils flottent et ce, en moins de quelques minutes.

De façon surprenante, les inventeurs ont découvert qu'un générateur de gaz est essentiel à la formulation d'un tel comprimé matriciel. Cependant, il a été observé que plus la quantité de générateur de gaz diminue jusqu'à 5% en poids du poids total du comprimé, plus les propriétés de flottaison (temps de flottaison) sont améliorées.

En outre, les inventeurs ont découvert qu'un superdésintégrant est essentiel à la formulation d'un tel comprimé matriciel.

D'autre part, les inventeurs ont découvert que la présence de 10% en poids du poids total du comprimé d'alginate de sodium est défavorable à la flottaison.

La présente invention a pour objet une composition pharmaceutique telle que définie dans les revendications, qui est sous forme de comprimé à résidence gastrique avec flottaison améliorée, contenant du baclofène, utilisable pour une administration une fois par jour ou deux fois par jour selon la matrice utilisée. Ces compositions remédient aux inconvénients mentionnés plus haut.

L'invention est caractérisée par le fait qu'au contact avec le liquide gastrique, la composition flotte rapidement. Ceci permet d'assurer un temps de séjour plus long du comprimé dans l'estomac et d'assurer que la plus grande proportion de principe actif contenu dans la composition pharmaceutique, soit libérée et absorbée dans la portion haute du tractus.

La présente invention a pour objet un comprimé pharmaceutique matriciel tel que défini dans les revendications, qui est administrable par voie orale une à deux fois par jour à libération contrôlée de Baclofène par rétention gastrique comprenant des granules de Baclofène et d'un ou plusieurs diluants au sein d'une matrice comprenant au moins un agent gélifiant, un agent générateur de gaz et un superdésintégrant.

L'agent générateur de gaz génère des bulles de gaz emprisonnées dans le superdésintégrant qui, lui, gonfle au contact du liquide gastrique jusqu'à deux fois son volume initial. Les agents gélifiants génèrent un réseau de libération prolongée du principe actif.

La formulation de l'invention est particulièrement avantageuse par le temps extrêmement court pour se mettre à flotter. En effet, si la formulation met trop de temps à se mettre à flotter, elle est vidangée. La formulation de l'invention présente une flottaison améliorée.

On entend par temps de mise en flottaison, le temps au bout duquel les comprimés remontent en surface du milieu (temps chronométré par observation visuelle) lors du test de dissolution type II sous agitation par pales (à une vitesse de rotation de 100rpm) tel que décrit dans la pharmacopée européenne 7ème édition (monographie 2.9.3). Les comprimés restent ensuite en surface tout le temps du test de dissolution.

Par flottaison améliorée, on entend un temps de mise en flottaison inférieur à 30 minutes en milieu gastrique nourri (pH entre 3 et 5) et inférieur à 10 minutes en milieu gastrique à jeun (pH entre 1 et 2).

Le terme « libération prolongée » dans la présente demande est utilisé pour désigner un profil de libération du principe actif modifié par rapport à celui qu'aurait présenté le principe actif seul dans un système à libération immédiate.

De préférence, la libération prolongée selon l'invention est comprise entre 4 heures et 24 heures selon la composition de la matrice polymère.

On entend par « comprimé matriciel » une composition pharmaceutique pour administration orale renfermant une substance active dispersée uniformément dans un ou des excipients qui, après compression, permettent la formation d'une matrice capable de freiner la libération du principe actif.

Des diluants appropriés pour la granulation du Baclofène sont les dérivés cellulosiques tels que la cellulose microcristalline comme l'Avicel PH102, les polyols comme le mannitol. De préférence, le Baclofène est granulé avec du mannitol.

Par « agent gélifiant » ou « agent contrôlant la libération », on entend les gommes d'origine végétale de nature glucidique, les polysaccharides linéaires (extraits d'algues), linéaires substitués (galactomannanes, gomme xanthane), les polyéthylène glycol (oxydes de polyéthylène), la méthylcellulose, l'hydroxypropylcellulose. Cet agent gélifiant a pour fonction dans la présente invention de former un réseau capable de retenir la formation de gaz qui est généré au contact du comprimé avec le milieu gastrique et d'assurer la libération contrôlée. Le comprimé selon l'invention peut aussi comprendre un mélange d'agents gélifiants.

Le comprimé selon l'invention comprend de 5 à 50% en poids d'agent gélifiant par rapport au poids total du comprimé, de préférence de 8 à 40 %, de manière particulièrement préférée de 10 à 30% en poids par rapport au poids total du comprimé.

Selon l'invention les agents gélifiants préférés sont la gomme Xanthane, les oxydes de polyéthylène (PEO), d'un poids moléculaire d'environ 4 000 000 g/mol (Polyox^{™} WSR301), d'un poids moléculaire d'environ 900 000 g/mol (Polyox^{™} WSR1105), d'un poids moléculaire d'environ 700 000 g/mol (Polyox^{™} WSR303).

Le comprimé selon l'invention comprend moins de 5% en poids du poids total du comprimé d'alginate de sodium.

De manière particulièrement préférée, le comprimé selon l'invention ne comprend pas d'alginate de sodium.

Par « agent générateur de gaz » selon l'invention, on entend un composé qui génère du gaz lorsqu'il est en contact avec un milieu acide tel que le suc gastrique. L'agent générateur de gaz selon l'invention est choisi parmi le groupe qui comprend les carbonates tels que le carbonate de sodium et le carbonate glycine sodium, les bicarbonates tels que le bicarbonate de sodium (NaHCO₃), le bicarbonate de potassium, les sulfites tels que le sulfite de sodium, le bisulfite de sodium, le métabisulfite de sodium, et les mélanges de ceux-ci.

De préférence, l'agent générateur de gaz est le bicarbonate de sodium.

Le comprimé selon l'invention comprend de 3 à 15% en poids de générateur de gaz par rapport au poids total du comprimé, de préférence 4 à 10%, de manière particulièrement préférée 5 % en poids par rapport au poids total du comprimé.

Par superdésintégrant on entend les polymères capables de capter rapidement le liquide environnant, ils comprennent par exemple les polymères cellulosiques réticulés, la carboxyméthylcellulose réticulée ou la croscarmellose, la crospovidone ou polyvinylpolypyrrolidone réticulée qui est un homopolymère réticulé de N-vinyl-2-pyrrolidinone. A titre d'exemple on peut citer la crospovidone commercialisée sous le nom de Kollidon^{®}CL ou aussi sous le nom de Polyplasdone^{™}.

Le comprimé selon l'invention comprend de 1 à 30% en poids de superdésintégrant par rapport au poids total du comprimé, de préférence de 1 à 20%, de 2 à 15%, de manière particulièrement préférée de 3 à 10 % en poids par rapport au poids total du comprimé.

Selon l'invention, le superdésintégrant préféré est la croscarmellose, la crospovidone ou polyvinylpolypyrrolidone réticulée qui est un homopolymère réticulé de N-vinyl-2-pyrrolidinone, la crospovidone commercialisée sous le nom de Kollidon^{®}CL ou aussi sous le nom de Polyplasdone^{™}.

Selon un mode de réalisation préféré de l'invention, le comprimé selon l'invention comprend l'agent gélifiant et le générateur de gaz dans un rapport en poids agent gélifiant:générateur de gaz de 1:10 à 10:1.

De manière particulièrement préférée, le comprimé selon l'invention comprend un superdésintégrant qui est la croscarmellose ou la crospovidone dans des proportions comprises entre 3% et 8% en poids du poids total du comprimé, un agent générateur de gaz qui est le bicarbonate de sodium dans des proportions comprises entre 4% et 8 % en poids du poids total du comprimé, un agent gélifiant qui est soit la gomme xanthane, soit le polyox^{™} dans des proportions comprises entre 10 % et 30 % en poids du poids total du comprimé.

En règle générale, la matrice du comprimé de l'invention comprend également un ou plusieurs diluants choisi(s) parmi les dérivés cellulosiques tels que la cellulose microcristalline, les polyols comme le mannitol, le carbonate de calcium.

La matrice du comprimé de l'invention peut ainsi comprendre de 5 à 75 %, de préférence de 10 à 60%, de manière particulièrement préférée de 15 à 50% en poids par rapport au poids total du comprimé d'un ou plusieurs diluants.

La matrice du comprimé de l'invention peut ainsi comprendre tout excipient connu pour moduler la libération contrôlée du principe actif. Ainsi, l'homme du métier saura ajuster la composition pour qu'elle soit adaptée à une administration une fois par jour ou deux fois par jour.

Le comprimé de l'invention peut comprendre également tout excipient adéquat et nécessaire à la fabrication du comprimé, tels que des lubrifiants comme le stéarate de magnésium ou du fumarate de stéaryle sodique et des agents d'écoulements tels que le talc ou la silice.

Selon l'invention lorsque les compositions pharmaceutiques comprennent un agent d'écoulement et /ou un lubrifiant, l'agent d'écoulement ou le lubrifiant est présent dans des proportions allant de 0,2 % à 2%, de préférence dans des proportions allant de 0,5 à 1,5% en poids du poids total du comprimé.

La composition pharmaceutique selon l'invention peut comprendre tout excipient additionnel classique de formulation ainsi que des arômes et des colorants.

A la composition pharmaceutique selon l'invention on peut en outre appliquer un enrobage en matériaux polymériques ayant pour but une simple protection ayant pour but une simple protection à l'humidité ou d'apporter une coloration pour la différenciation des dosages ou bien une modulation de la cinétique de libération du principe actif à partir de la matrice polymérique selon les techniques connues de l'homme du métier.

Les comprimés selon l'invention sont obtenus par les techniques bien connues de l'homme du métier.

En particulier, les granules sont obtenus par granulation humide par mise en présence du Baclofène avec un ou plusieurs diluants et optionnellement en présence d'un liant. Après séchage, ces granules sont uniformément mélangés avec les excipients constituant la matrice et l'ensemble est comprimé.

La formulation selon l'invention comprend de 10 mg à 300 mg de Baclofène par unité de dose, de préférence de 30 mg à 150 mg, pouvant être prise une à deux fois par jour.

L'invention concerne également un comprimé selon l'invention pour son utilisation en tant que médicament, de préférence par voie orale en prise unique une fois par jour ou bien en deux prises par jour.

Un autre objet de l'invention concerne un comprimé selon l'invention pour son utilisation dans le traitement de la dépendance à l'alcool ou du maintien du sevrage des alcooliques.

### Exemples

### 1. EXEMPLE DE COMPOSITION SELON LE BREVET EP 1745775 (EXEMPLE COMPARATIF)

Dans cet exemple, la composition des comprimés respecte les quantités décrites dans le brevet EP 1745775.

**Tableau 1 : composition centésimale analogue à celle décrite dans le brevet EP 1745775**

| **Phase** | **Composant** | **mg/unité** | **% p/p** |
|---|---|---|---|
| Granulation humide | Baclofène | 30,0 | 6,0 |
| | Gomme Xanthane (Vanzan^{R} NF) | 50,0 | 10,0 |
| | cellulose microcristalline et carboxyméthylcellulose sodium (Avicel ^{R} CL 611) | 50,0 | 10,0 |
| | Mannitol SD200 | 100,0 | 20,0 |
| | HPMC 603 (Pharmacoat^{R} 603) | 15,0 | 3,0 |
| Mélange | Mannitol SD 200 | 146,5 | 29,3 |
| | Bicarbonate de sodium | 101,0 | 20,2 |
| | Silice précipitée (Syloid^{R} 244FP) | 2,5 | 0,5 |
| Lubrification | Stéarate de Magnésium | 5,0 | 1,0 |
| | Total | 500,0 | 100,0 |

Le baclofène est granulé avec la gomme Xanthane, de l'Avicel CL611, du mannitol SD200 et de l'HPMC selon un procédé de granulation humide dans un mélangeur granulateur à haut cisaillement avec ajout d'un mélange eau / éthanol dans un ratio 1 : 1 % (p/p). Après séchage et calibrage sur un tamis oscillant de maille de 425µm, le principe actif granulé est mélangé avec les excipients du mélange dans un mélangeur multidirectionnel (Turbula 2L) pendant 10 minutes puis lubrifié avec le stéarate de magnésium pendant 1 minute supplémentaire. Le mélange final est ensuite comprimé sur une presse rotative (SVIAC PR12) équipée de poinçons ronds de diamètre 12 mm et d'un système d'alimentation forcée.

Les comprimés sont produits à une force de compression de 18kN et présentent une dureté de 105N.

Le tableau 2 présente les résultats de dissolution dans 500 ml de milieu à pH 4.5, selon le test USP II (agitation par pales à 100rpm avec présence d'un disque).

**Tableau 2 : test de dissolution du comprimé**

| **Temps (h)** | **% dissous** | **CV (%)** |
|---|---|---|
| 1 | 43,00 | 12,26 |
| 2 | 69,25 | 4,65 |
| 3 | 75,11 | 3,97 |
| 4 | 80,08 | 2,28 |
| 6 | 87,87 | 2,42 |
| 12 | 100,88 | 0,88 |

Lors du test de dissolution, la flottaison des comprimés a été observée au bout de 1h45 minutes.

### 2. EXEMPLE DE COMPOSITION AVEC ALGINATE DE SODIUM (EXEMPLE COMPARATIF)

Dans cet exemple, l'alginate de sodium a été ajouté à la composition.

**Tableau 3 : composition centésimale des comprimés comprenant de l'alginate de sodium**

| **Phase** | **Composant** | **mg/unité** | **% (p/p)** |
|---|---|---|---|
| Granulation humide | Baclofène | 30,0 | 6,0 |
| | Mannitol SD200 | 120,0 | 24,0 |
| Mélange | Gomme Xanthane (Vanzan^{R} NF) | 37,5 | 7,5 |
| | Alginate de sodium (Keltone^{R} LVCR) | 50,0 | 10,0 |
| | Crospovidone (Polyplasdone^{™} Ultra) | 37,5 | 7,5 |
| | Bicarbonate de sodium | 100,0 | 20,0 |
| | Cellulose microcristalline PH102 (Avicel^{R} PH102) | 65,0 | 13,0 |
| | Calcium Phosphate, Dibasic Dihydrate (Emcompress^{R} ) | 50,0 | 10,0 |
| | Silice précipitée (Syloid^{R} 244FP) | 5,0 | 1,0 |
| Lubrification | Stéarate de Magnésium | 5,0 | 1,0 |
| | Total | 500,0 | 100,0 |

Le baclofène est granulé avec le mannitol SD200 selon un procédé de granulation humide dans un mélangeur granulateur à haut cisaillement (Diosna P/VAC 10) avec ajout d'eau purifiée. Après séchage et calibrage sur un tamis oscillant de maille de 425 µ*m*, le principe actif granulé est mélangé avec les excipients du mélange (sauf le lubrifiant) dans un mélangeur multidirectionnel (Turbula 2L) 10 minutes, puis lubrifié avec le stéarate de magnésium pendant 1 minute supplémentaire. Le mélange final est ensuite comprimé sur une presse rotative (Sviac PR12) équipée de poinçons ronds de diamètre 12 mm et d'un système d'alimentation forcée. Les comprimés produits à une force de compression de 20kN présentent une dureté moyenne de 103N.

Le tableau 4 présente les résultats de dissolution dans 500ml de milieu tampon pH 4.5, selon le test USP II (agitation par pales à 100rpm avec présence d'un disque).

**Tableau 4 : test de dissolution dans 500 ml de milieu a ph 4,5**

| **Temps (h)** | **% dissous** | **CV (%)** |
|---|---|---|
| 1 | 25,35 | 9,58 |
| 2 | 44,45 | 2,02 |
| 4 | 57,37 | 1,20 |
| 8 | 70,91 | 2,56 |
| 10 | 76,35 | 0,83 |
| 16 | 89,82 | 0,71 |

La flottaison des comprimés lors du test de dissolution a été observée au bout de 55 minutes à 75 minutes. L'alginate à 10% p/p ralentit le temps de flottaison.

### 3. EXEMPLE DE COMPOSITIONS SANS BICARBONATE DE SODIUM (EXEMPLE COMPARATIF)

Dans cet exemple, le mélange est préparé de la même manière que dans l'exemple 2. Il est ensuite comprimé sur une presse rotative (Sviac PR12) équipée de poinçons ronds de diamètre 12 mm et d'un système d'alimentation forcée. La composition centésimale est présentée au tableau 5.

**Tableau 5 : composition centésimale des comprimés sans bicarbonate de sodium**

| **Phase** | **Composant** | **mg/unité** | **% (p/p)** |
|---|---|---|---|
| Granulation humide | Baclofène | 30,0 | 6,0 |
| | Mannitol SD200) | 120,0 | 24,0 |
| Mélange | Gomme Xanthane (Vanzan^{R} NF) | 125,0 | 25,0 |
| | Crospovidone (Polyplasdone^{™} Ultra) | 100,0 | 20,0 |
| | Bicarbonate de sodium | - | - |
| | Cellulose microcristalline PH102 (Avicel^{R} PH 102) | 100,0 | 20,0 |
| | Mannitol SD200 | 15,0 | 3,0 |
| | Silice précipitée (Syloid^{R} 244FP) | 5,0 | 1,0 |
| Lubrification | Stéarate de Magnésium | 5,0 | 1,0 |
| | Total | 500,0 | 100,0 |

Les comprimés sont produits à une force de compression de 16kN et présentent une dureté moyenne de 147N. Le tableau 6 présente les résultats de dissolution dans 500ml de milieu tampon pH 4.5, selon le test USP II (agitation par pales à 100rpm avec présence d'un disque).

**Tableau 6 : test de dissolution dans 500 ml de milieu à pH 4,5**

| **Temps (h)** | **% dissous** | **CV (%)** |
|---|---|---|
| 1 | 12,26 | 2,10 |
| 2 | 20,25 | 2,35 |
| 4 | 33,98 | 1,32 |
| 8 | 65,84 | 14,41 |
| 12 | 81,76 | 12,77 |
| 20 | 93,32 | 6,18 |

Les comprimés ne flottent à aucun moment pendant le test de dissolution. La flottaison n'est jamais atteinte. La formulation de l'exemple 3 ne résout donc pas le problème technique.

### 4. EXEMPLE AVEC 5% DE GELIFIANT (EXEMPLE COMPARATIF)

Dans cet exemple, le mélange est préparé de la même manière que dans l'exemple 2. Il est ensuite comprimé sur une presse rotative (Sviac PR12) équipée de poinçons ronds de diamètre 12 mm et d'un système d'alimentation forcée.

La composition est présentée au tableau 7.

**Tableau 7 :composition centésimale de comprimé contenant 5% de gélifiant**

| **Phase** | **Composant** | **mg/unité** | **% (p/p)** |
|---|---|---|---|
| Granulation humide | Baclofène | 30,0 | 6,0 |
| | Mannitol SD200 | 120,0 | 24,0 |
| Mélange | Gomme Xanthane (Vanzan^{R} NF) | 25,0 | 5,0 |
| | Crospovidone (Polyplasdone^{™} Ultra) | 75,0 | 15,0 |
| | Bicarbonate de sodium | 117,5 | 23,5 |
| | Cellulose microcristalline PH102 (Avicel^{R} PH102) | 95,5 | 19,1 |
| | Mannitol SD200 | 27,0 | 5,4 |
| | Silice précipitée (Syloid^{R} 244FP) | 5,0 | 1,0 |
| Lubrification | Stéarate de Magnésium | 5,0 | 1,0 |
| | Total | 500,0 | 100,0 |

Les comprimés sont produits à une force de compression de 21KN et présentent une dureté moyenne de 125N. Les résultats du test de dissolution dans le tampon pH 4,5 selon le test USP II (agitation par pales à 100rpm avec présence d'un disque) sont présentés dans le tableau 8.

**Tableau 8 : test de dissolution dans 500 ml de milieu à pH 4,5**

| **Temps (h)** | **% dissous** | **CV (%)** |
|---|---|---|
| 0,5 | 74,58 | 1,65 |
| 1 | 81,08 | 1,90 |
| 2 | 86,79 | 1,14 |
| 3 | 87,86 | 0,25 |
| 6 | 88,10 | 0,43 |

La flottaison des comprimés a été observée lors du test de dissolution au bout de 30 secondes.

### 5. EXEMPLE AVEC 50% DE GELIFIANT (EXEMPLE COMPARATIF)

Dans cet exemple, le mélange est préparé de la même manière que dans l'exemple 2. Il est ensuite comprimé sur une presse rotative (Sviac PR12) équipée de poinçons oblongs de dimensions 19 x 7, 5 mm et d'un système d'alimentation forcée. La composition centésimale est présentée au tableau 9.

**Tableau 9 : composition centésimale des comprimés comprenant 50 % de gélifiant.**

| **Phase** | **Composant** | **mg/unité** | **%** |
|---|---|---|---|
| Granulation humide | Baclofène | 30,0 | 4,6 |
| | Mannitol SD200 | 133,1 | 20,5 |
| Mélange | HPMC K100M (Methocel^{™} K100M) | 325,0 | 50,0 |
| | Crospovidone (Polyplasdone^{™} Ultra) | 65,0 | 10,0 |
| | Bicarbonate de sodium | 32,5 | 5,0 |
| | Cellulose microcristalline PH102 (Avicel^{R} PH102) | 51,4 | 7,9 |
| | Silice précipitée (Syloid^{R} 244FP) | 6,5 | 1,0 |
| Lubrification | Stéarate de Magnésium | 6,5 | 1,0 |
| | Total | 650,0 | 100,0 |

Les comprimés sont produits à une force de compression de 10kN et présentent une dureté moyenne de 133N. Le tableau 10 présente les résultats de dissolution dans 500ml de milieu tampon pH 1.2, selon le test USP II (agitation par pales à 100rpm avec présence d'un disque).

**Tableau 10 : test de dissolution dans 500 ml de milieu à pH 1,2**

| **Temps (h)** | **% dissous** | **CV (%)** |
|---|---|---|
| 1 | 18,17 | 1,78 |
| 2 | 26,95 | 3,90 |
| 4 | 40,93 | 7,57 |
| 8 | 61,63 | 10,65 |
| 12 | 76,31 | 11,02 |
| 24 | 97,45 | 5,18 |

Lors du test de dissolution, la flottaison des 6 comprimés a été observée au bout de 4 à 8 min.

### 6. EXEMPLE AVEC 30% DE GELIFIANT (EXEMPLE COMPARATIF)

Dans cet exemple, le mélange est préparé de la même manière que dans l'exemple 2. Il est ensuite comprimé sur une presse rotative (Sviac PR12) équipée de poinçons ronds de diamètre 12 mm et d'un système d'alimentation forcée. La composition est présentée au tableau 11.

**Tableau 11 : composition centésimale du comprimé comprenant 30% de gélifiant**

| **Phase** | **Composant** | **mg/unité** | **%** |
|---|---|---|---|
| Granulation humide | Baclofène | 30,0 | 7,1 |
| | Mannitol SD200 | 120,0 | 28,2 |
| Mélange | HPMC K15M (Methocel^{™} K15M) | 127,5 | 30,0 |
| | Crospovidone (Polyplasdone^{™} ultra) | 63,7 | 15,0 |
| | Bicarbonate de sodium | 21,3 | 5,0 |
| | Cellulose microcristalline PH102 (Avicel^{R} PH102) | 54,0 | 12,7 |
| | Silice précipitée (Syloid^{R} 244FP) | 4,3 | 1,0 |
| Lubrification | Stéarate de Magnésium | 4,3 | 1,0 |
| | Total | 425,0 | 100,0 |

Les comprimés sont produits à une force de compression de 16kN et présentent une dureté moyenne de 116N. Le tableau 12 présente les résultats de dissolution dans 500ml de milieu tampon pH 1.2, selon le test USP II (agitation par pales à 100rpm avec présence d'un disque).

**Tableau 12 : test de dissolution dans 500 ml de milieu à pH 1,2**

| **Temps (h)** | **% dissous** | **CV (%)** |
|---|---|---|
| 1 | 25,87 | 1,86 |
| 2 | 37,69 | 1,47 |
| 4 | 54,70 | 1,69 |
| 8 | 76,72 | 1,72 |
| 16 | 94,41 | 1,37 |

La flottaison des comprimés lors du test de dissolution a été observée au bout de 15 secondes.

### 7. EXEMPLE AVEC 20% DE GELIFIANT

Dans cet exemple, le mélange est préparé de la même manière que dans l'exemple 2. Il est ensuite comprimé sur une presse rotative (Sviac PR12) équipée de poinçons ronds de diamètre 12 mm et d'un système d'alimentation forcée. La composition est présentée au tableau 13.

**Tableau 13 : composition centésimale du comprimé comprenant 20% de gélifiant**

| **Phase** | **Composant** | **mg/unité** | **%** |
|---|---|---|---|
| Granulation humide | Baclofène | 30,0 | 7,1 |
| | Mannitol SD200 | 120,0 | 28,2 |
| Mélange | Polyéthylène oxyde (Polyox WSR 303) | 85,0 | 20,0 |
| | Crospovidone (Polyplasdone^{™} ultra) | 63,7 | 15,0 |
| | Bicarbonate de sodium | 21,3 | 5,0 |
| | Cellulose microcristalline PH102 (Avicel^{R} PH102) | 96,4 | 22,7 |
| | Silice précipitée (Syloid^{R} 244FP) | 4,3 | 1,0 |
| Lubrification | Stéarate de Magnésium | 4,3 | 1,0 |
| | Total | 425,0 | 100,0 |

Les comprimés sont produits à une force de compression de 16kN présentent une dureté moyenne de 116N. Le tableau 14 présente les résultats de dissolution dans 500ml de milieu tampon pH 4.5, selon le test USP II (agitation par pales à 100rpm avec présence d'un disque).

**Tableau 14 : test de dissolution dans 500 ml de milieu à pH 4,5**

| **Temps (h)** | **% dissous** | **CV (%)** |
|---|---|---|
| 1 | 17,77 | 6,83 |
| 2 | 24,94 | 5,25 |
| 4 | 37,44 | 3,06 |
| 8 | 60,02 | 2,40 |
| 16 | 84,12 | 1,45 |

La flottaison des comprimés lors du test de dissolution a été observée au bout de 6 minutes.

### 8. EXEMPLE AVEC 10% DE GELIFIANT (EXEMPLE COMPARATIF)

Dans cet exemple, le mélange est préparé de la même manière que dans l'exemple 2. Il est ensuite comprimé sur une presse rotative (Sviac PR12) équipée de poinçons ronds de diamètre 12 mm et d'un système d'alimentation forcée. La composition est présentée au tableau 15.

**Tableau 15 : composition centésimale du comprimé comprenant 10% de gélifiant**

| **Phase** | **Composant** | **mg/unité** | **%** |
|---|---|---|---|
| Granulation humide | Baclofène | 30,0 | 7,1 |
| | Mannitol SD200 | 120,0 | 28,2 |
| Mélange | HPMC K100M (Methocel^{™} K100M) | 42,5 | 10,0 |
| | Crospovidone (Polyplasdone^{™} Ultra) | 21,3 | 5,0 |
| | Bicarbonate de sodium | 21,3 | 5,0 |
| | Cellulose microcristalline PH102 (Avicel^{R} PH102) | 160,3 | 37,7 |
| | Carbonate de calcium | 21,3 | 5,0 |
| | Silice précipitée (Syloid^{R} 244FP) | 4,3 | 1,0 |
| Lubrification | Stéarate de Magnésium | 4,3 | 1,0 |
| | Total | 425,0 | 100,0 |

Les comprimés sont produits à une force de compression de 15kN et présentent une dureté moyenne de 122N. Le tableau 16 présente les résultats de dissolution dans 500ml de milieu tampon pH 4.5, selon le test USP II (agitation par pales à 100rpm avec présence d'un disque).

**Tableau 16 : test de dissolution dans 500 ml de milieu à pH 4,5**

| **Temps (h)** | **% dissous** | **CV (%)** |
|---|---|---|
| 1 | 39,99 | 1,18 |
| 2 | 52,25 | 0,62 |
| 4 | 68,45 | 0,43 |
| 8 | 87,17 | 0,59 |
| 16 | 96,87 | 1,36 |

La flottaison des comprimés lors du test de dissolution a été observée au bout de 20 secondes.

### 9. EXEMPLE AVEC CROSCARMELLOSE SODIQUE (EXEMPLE COMPARATIF)

Dans cet exemple, le mélange est préparé de la même manière que dans l'exemple 2. Il est ensuite comprimé sur une presse rotative (Sviac PR12) équipée de poinçons ronds de diamètre 12 mm et d'un système d'alimentation forcée. La composition est présentée au tableau 17.

**Tableau 17 : composition centésimale du comprimé comprenant de la croscaramellose sodique**

| **Phase** | **Composant** | **mg/unité** | **%** |
|---|---|---|---|
| Granulation humide | Baclofène | 30,0 | 6,0 |
| | Mannitol SD200 | 120,0 | 24,0 |
| Mélange | Gomme Xanthane (Vanzan^{R} NF) | 40,0 | 8,0 |
| | Croscarmellose sodique (AcDiSol^{R}) | 60,0 | 12,0 |
| | Bicarbonate de sodium | 117,5 | 23,5 |
| | Cellulose microcristalline PH102 (Avicel^{R} PH 102) | 95,5 | 19,1 |
| | Mannitol SD200 | 27,0 | 5,4 |
| | Silice précipitée (Syloid^{R} 244FP) | 5,0 | 1,0 |
| Lubrification | Stéarate de Magnésium | 5,0 | 1,0 |
| | Total | 500,0 | 100,0 |

Les comprimés sont produits à une force de compression de 22kN et présentent une dureté moyenne de 141N. Le tableau 18 présente les résultats de dissolution dans 500ml de milieu tampon pH 4.5, selon le test USP II (agitation par pales à 100rpm avec présence d'un disque).

**Tableau 18 : test de dissolution dans 500 ml de milieu à pH 4,5**

| **Temps (h)** | **% dissous** | **CV (%)** |
|---|---|---|
| 1 | 43,74 | 4,82 |
| 2 | 52,30 | 3,38 |
| 4 | 63,92 | 2,47 |
| 6 | 71,97 | 1,40 |
| 12 | 90,76 | 0,34 |

La flottaison des comprimés lors du test de dissolution a été observée au bout de 30 secondes.

### 10. EXEMPLE AVEC 20% DE CROSPOVIDONE

Dans cet exemple, le mélange est préparé de la même manière que dans l'exemple 2. Il est ensuite comprimé sur une presse rotative (Sviac PR12) équipée de poinçons ronds de diamètre 12 mm et d'un système d'alimentation forcée. La composition est présentée au tableau 19.

**Tableau 19 : composition centésimale du comprimé comprenant 20 % de crospovidone**

| **Phase** | **Composant** | **mg/unité** | **%** |
|---|---|---|---|
| Granulation humide | Baclofène | 30,0 | 6,0 |
| | Mannitol SD200) | 120,0 | 24,0 |
| Mélange | Gomme Xanthane (Vanzan^{R} NF)) | 125,0 | 25,0 |
| | Crospovidone (Polyplasdone^{™} Ultra) | 100,0 | 20,0 |
| | Bicarbonate de sodium | 25,0 | 5,0 |
| | Cellulose microcristalline PH102 (Avicel^{R} PH 102) | 75,0 | 15,0 |
| | Mannitol SD200 | 15,0 | 3,0 |
| | Silice précipitée (Syloid^{R} 244FP) | 5,0 | 1,0 |
| Lubrification | Stéarate de Magnésium | 5,0 | 1,0 |
| | Total | 500,0 | 100,0 |

Les comprimés sont produits à une force de compression de 18kN et présentent une dureté moyenne de 121N. Le tableau 19 présente les résultats de dissolution dans 500ml de milieu tampon pH 4.5, selon le test USP II (agitation par pales à 100rpm avec présence d'un disque).

**Tableau 20 : test de dissolution dans 500 ml de milieu à pH 4,5**

| **Temps (h)** | **% dissous** | **CV (%)** |
|---|---|---|
| 1 | 10,16 | 1,08 |
| 2 | 16,67 | 0,97 |
| 4 | 28,28 | 1,04 |
| 8 | 49,11 | 0,88 |
| 12 | 67,63 | 1,43 |
| 20 | 96,81 | 1,01 |

La flottaison des comprimés lors du test de dissolution a été observée au bout de 9 minutes.

### 11. EXEMPLE AVEC 90MG DE PRINCIPE ACTIF (EXEMPLE COMPARATIF)

Dans cet exemple, le mélange est préparé de la même manière que dans l'exemple 2. Il est ensuite comprimé sur une presse rotative (Sviac PR12) équipée de poinçons ronds de diamètre 12 mm et d'un système d'alimentation forcée. La composition est présentée au tableau 21.

**Tableau 21 : composition centésimale du comprimé comprenant 90 mg de principe actif.**

| **Phase** | **Composant** | **mg/unité** | **%** |
|---|---|---|---|
| Granulation humide | Baclofène | 90,0 | 18,0 |
| | Mannitol SD200 | 60,0 | 12,0 |
| Mélange | HPMC K100M (Methocel^{™} K100M) | 150,0 | 30,0 |
| | Crospovidone (Polyplasdone^{™} Ultra) | 25,0 | 5,0 |
| | Bicarbonate de sodium | 25,0 | 5,0 |
| | Cellulose microcristalline PH102 (Avicel^{R} PH102) | 90,0 | 18,0 |
| | Carbonate de calcium | 50,0 | 10,0 |
| | Silice précipitée (Syloid^{R} 244FP) | 5,0 | 1,0 |
| Lubrification | Stéarate de Magnésium | 5,0 | 1,0 |
| | Total | 500,0 | 100,0 |

Les comprimés sont produits à une force de compression de 14kN et présentent une dureté moyenne de 115N. Le tableau 22 présente les résultats de dissolution dans 500ml de milieu tampon pH 1.2, selon le test USP II (agitation par pales à 100rpm avec présence d'un disque).

**Tableau 22 : test de dissolution dans 500 ml de milieu à pH 1,2**

| **Temps (h)** | **% dissous** | **CV (%)** |
|---|---|---|
| 1 | 15,92 | 4,19 |
| 2 | 23,78 | 1,62 |
| 4 | 35,56 | 2,63 |
| 8 | 53,00 | 2,53 |
| 16 | 80,34 | 2,29 |
| 24 | 95,45 | 1,87 |

La flottaison des comprimés lors du test de dissolution en pH 1.2 a été observée au bout de 30 secondes.

### 12. EXEMPLE AVEC PELLICULAGE COLORE (EXEMPLE COMPARATIF)

Dans cet exemple, le mélange est préparé de la même manière que dans l'exemple 2. Il est ensuite comprimé sur une presse rotative (Sviac PR12) équipée de poinçons ronds de diamètre 12 mm et d'un système d'alimentation forcée.

**Tableau 23 : composition centésimale du comprimé comprenant un pelliculage coloré**

| **Phase** | **Composant** | **mg/unité** | **%** |
|---|---|---|---|
| Granulation humide | Baclofène | 30,0 | 5,8 |
| | Mannitol SD200 | 120,0 | 23,3 |
| Mélange | HPMC K100M (Methocel^{™} K100M) | 150,0 | 29,1 |
| | Crospovidone (Polyplasdone^{™} Ultra) | 25,0 | 4,8 |
| | Bicarbonate de sodium | 25,0 | 4,8 |
| | Cellulose microcristalline PH102 (Avicel^{R} PH102) | 90,0 | 17,5 |
| | Carbonate de calcium | 50,0 | 9,7 |
| | Silice précipitée (Syloid^{R} 244FP) | 5,0 | 1,0 |
| Lubrification | Stéarate de Magnésium | 5,0 | 1,0 |
| Pelliculage coloré | Opadry II 85F230062 | 15,5 | 3,0 |
| | Total | 515,5 | 100,0 |

Les comprimés sont produits à une force de compression de 12kN et présentent une dureté moyenne de 88N. Les comprimés sont ensuite pelliculés dans une turbine perforée (Labcoat M O'hara) avec 3% d'Opadry II pour la coloration. Après pelliculage, la dureté moyenne des comprimés est de 140N.

Le tableau 24 présente les résultats de dissolution dans 500ml de milieu tampon pH 1.2, selon le test USP II (agitation par pales à 100rpm avec présence d'un disque).

**Tableau 24 : test de dissolution dans 500 ml de milieu à pH 1,2**

| **Temps (h)** | **% dissous** | **CV (%)** |
|---|---|---|
| 1 | 15,94 | 1,74 |
| 2 | 24,90 | 1,06 |
| 4 | 37,88 | 1,18 |
| 8 | 56,40 | 0,55 |
| 16 | 85,78 | 0,74 |
| 24 | 97,10 | 1,54 |

Le temps de flottaison relevé lors du test de dissolution en pH 1.2 est de 4 minutes. Le temps de flottaison relevé dans le milieu tampon pH 4.5 dans les mêmes conditions d'agitation est de 18 minutes.

### 13. EXEMPLE AVEC 150MG DE PRINCIPE ACTIF ET UN COUPLE DE GELIFIANTS (EXEMPLE COMPARATIF)

**Tableau 25 : composition centésimale du comprimé comprenant 150 mg de principe actif et un couple de gélifiants**

| **Phase** | **Composant** | mg/unité | % |
|---|---|---|---|
| Granulation humide | Baclofène | 150,0 | 21,4 |
| | Mannitol SD200 | 93,75 | 13,4 |
| | HPMC 603 (Pharmacoat 603) | 6,25 | 0,9 |
| Mélange | HPMC K100M (Methocel^{™} K100M) | 70,0 | 10,0 |
| | HPMC K4M (Methocel^{™} K4M) | 35,0 | 5,0 |
| | Crospovidone (Polyplasdone^{™} Ultra) | 21,3 | 3,0 |
| | Bicarbonate de sodium | 21,3 | 3,0 |
| | Cellulose microcristalline PH200 (Avicel^{R} PH200) | 267,1 | 38,2 |
| | Carbonate de calcium | 21,3 | 3,0 |
| | Silice précipitée (Syloid^{R} 244FP) | 7,0 | 1,0 |
| Lubrification | Stéarate de Magnésium | 7,0 | 1,0 |
| | Total | 700,0 | 100,0 |

Dans cet exemple, le baclofène est granulé avec le mannitol SD 200 selon un procédé de granulation humide dans un mélangeur granulateur à haut cisaillement (Diosna P/VAC 10) avec ajout d'HPMC 603 et d'eau purifiée. Après séchage et calibrage sur un tamis oscillant de maille 425 µm, le principe actif granulé est mélangé aux excipients de la même manière que dans l'exemple 2. Il est ensuite comprimé sur une presse rotative (Sviac PR12) équipée de poinçons oblongs de dimensions 19x7,5 mm et d'un système d'alimentation forcé.

Les comprimés produits à une force de compression de 8kN présentent une dureté moyenne de 124N. Le tableau ci-dessous présente les résultats de dissolution dans 500mL de milieu tampon pH 1.2, selon le test USP II (agitation par pales à 100rpm avec présence d'un disque).

**Tableau 26 : test de dissolution dans 500 ml de milieu à pH 1,2**

| Temps (h) | % dissout | CV (%) |
|---|---|---|
| 1 | 30,03 | 7,52 |
| 2 | 44,13 | 3,64 |
| 4 | 62,24 | 3,23 |
| 8 | 84,40 | 2,82 |
| 16 | 100,45 | 2,86 |
| 24 | 101,28 | 1,51 |

Le temps de flottaison relevé lors du test de dissolution en pH 1.2 est de 5 secondes. Le temps de flottaison relevé dans le milieu tampon pH 4.5 dans les mêmes conditions d'agitation est de 20 secondes.

### 14. EXEMPLE AVEC 150MG DE PRINCIPE ACTIF ET UN COUPLE DE GELIFIANTS (EXEMPLE COMPARATIF)

**Tableau 27 : composition centésimale du comprimé comprenant 150 mg de principe actif et un couple de gélifiants**

| **Phase** | **Composant** | mg/unité | % |
|---|---|---|---|
| Granulation humide | Baclofène | 150,0 | 21,4 |
| | Mannitol SD200 | 93,75 | 13,4 |
| | HPMC 603 (Pharmacoat 603) | 6,25 | 0,9 |
| Mélange | HPMC K100M (Methocel^{™} K100M) | 70,0 | 10,0 |
| | HPMC K4M (Methocel^{™} K4M) | 70,0 | 10,0 |
| | Crospovidone (Kollidon CL) | 35,0 | 5,0 |
| | Bicarbonate de sodium | 35,0 | 5,0 |
| | Cellulose microcristalline PH200 (Avicel^{R} PH200) | 205,0 | 29,3 |
| | Carbonate de calcium | 21,0 | 3,0 |
| | Silice précipitée (Syloid^{R} 244FP) | 7,0 | 1,0 |
| Lubrification | Stéarate de Magnésium | 7,0 | 1,0 |
| | Total | 700,0 | 100,0 |

Dans cet exemple, le baclofène est granulé avec le mannitol SD 200 selon un procédé de granulation humide dans un mélangeur granulateur à haut cisaillement (Diosna P/VAC 10) avec ajout d'HPMC 603 et d'eau purifiée. Après séchage et calibrage sur un tamis oscillant de maille 425 µm, le principe actif granulé est mélangé aux excipients de la même manière que dans l'exemple 2. Il est ensuite comprimé sur une presse rotative (Sviac PR12) équipée de poinçons oblongs de dimensions 18x9 mm et d'un système d'alimentation forcé.

Les comprimés produits à une force de compression de 21kN présentent une dureté moyenne de 202N. Le tableau ci-dessous présente les résultats de dissolution dans 500mL de milieu tampon pH 1.2, selon le test USP II (agitation par pales à 100rpm avec présence d'un disque).

**Tableau 28 : test de dissolution dans 500 ml de milieu à pH 1,2**

| Temps (h) | % dissout | CV (%) |
|---|---|---|
| 1 | 21,20 | 8,17 |
| 2 | 32,94 | 7,35 |
| 4 | 47,36 | 4,69 |
| 8 | 69,69 | 2,25 |
| 16 | 97,10 | 1,30 |
| 24 | 100,03 | 0,56 |

Le temps de flottaison relevé lors du test de dissolution en pH 1.2 est de 15 secondes. Le temps de flottaison relevé dans le milieu tampon pH 4.5 dans les mêmes conditions d'agitation est de 2 minutes.

### 15. EXEMPLE AVEC PELLICULAGE COLORE (EXEMPLE COMPARATIF)

**Tableau 29 : composition centésimale du comprimé avec pelliculage coloré**

| **Phase** | **Composant** | mg/unité | % |
|---|---|---|---|
| Granulation humide | Baclofène | 30,00 | 7,76 |
| | Mannitol SD200 | 43,13 | 11,16 |
| | HPMC 603 (Pharmacoat 603) | 1,88 | 0,49 |
| Mélange | HPMC K100M (Methocel^{™} K100M) | 75,00 | 19,40 |
| | Crospovidone (Kollidon CL) | 28,13 | 7,28 |
| | Bicarbonate de sodium | 28,13 | 7,28 |
| | Cellulose microcristalline PH102 (Avicel^{R} PH102) | 142,50 | 36,86 |
| | Carbonate de calcium | 18,75 | 4,85 |
| | Silice précipitée (Syloid^{R} 244FP) | 3,75 | 0,97 |
| Lubrification | Stéarate de Magnésium | 3,75 | 0,97 |
| Pelliculage coloré | Opadry II 85F240070 | 11,25 | 3,00 |
| | Total | 386,3 | 100,0 |

Dans cet exemple, le baclofène est granulé avec le mannitol SD 200 selon un procédé de granulation humide dans un mélangeur granulateur à haut cisaillement (Diosna P/VAC 10) avec ajout d'HPMC 603 et d'eau purifiée. Après séchage et calibrage sur un tamis oscillant de maille 425 µm, le principe actif granulé est mélangé aux excipients de la même manière que dans l'exemple 2. Il est ensuite comprimé sur une presse rotative (Sviac PR12) équipée de poinçons oblongs de dimensions 15x7 mm et d'un système d'alimentation forcé.

Les comprimés produits à une force de compression de 10kN présentent une dureté moyenne de 135N. Les comprimés sont ensuite pelliculés dans une turbine perforée (Labcoat M O'hara) avec 3% d'Opadry II pour une coloration. Après pelliculage, les comprimés ont une dureté moyenne de 166N.

Le tableau ci-dessous présente les résultats de dissolution dans 500mL de milieu tampon pH 1.2, selon le test USP II (agitation par pales à 100rpm avec présence d'un disque).

**Tableau 30: test de dissolution dans 500 ml de milieu à pH 1,2**

| Temps (h) | % dissout | CV (%) |
|---|---|---|
| 1 | 26,3 | 6,02 |
| 2 | 39,3 | 3,03 |
| 4 | 54,3 | 3,09 |
| 8 | 81,4 | 3,90 |
| 16 | 99,6 | 0,88 |
| 24 | 100,0 | 0,97 |

Le temps de flottaison relevé lors du test de dissolution en pH 1.2 est de 10 secondes. Le temps de flottaison relevé dans le milieu tampon pH 4.5 dans les mêmes conditions d'agitation est de 40 secondes.

### BIBLIOGRAPHIE

Dahan A, Miller J, M et Amidon G, L Prédiction of solubility and permeability class membership: provisional BCS Classification of the world's top oral drug [Revue] // The AAPS Journal, vol 11, n°4. - 2009. - pp. 740-746.
Davis S, S Formulation strategies for absorption windows [Revue] // Drug Discovery today, vol 10, n°4. - 2005.
Fasinu P [et al.] Diverse approaches for the enhancement of oral drug bioavailability [Revue] // Biopharmaceutics and drug disposition, vol 32. - 2011. - pp. 185-209.
Merino M [et al.] Evidence of a specialized transport mechanism for the intestinal absorption of baclofen [Revue] // Biopharmaceutics and Dreug disposition, vol 10. - 1989. - pp. 279-297.
Leisen C, C [et al.]Lipophilicities of baclofen ester prodrugs correlate with affinities to the ATP-dependant efflux pump P-glycoprotein: relevance for their permeation across the blood-brain barrier? [Revue] // Pharmaceutical Research, vol 20, n°5. - 2003. - pp. 772 -778.

## Revendications

1. Comprimé pharmaceutique matriciel administrable par voie orale une à deux fois par jour à libération contrôlée de Baclofène par rétention gastrique comprenant des granules obtenus par granulation humide de Baclofène, d'un ou plusieurs diluants choisis parmi les dérivés cellulosiques tels que la cellulose microcristalline et les polyols comme le mannitol, et éventuellement d'un liant au sein d'une matrice comprenant :
de 5 à 50% en poids d'au moins un agent gélifiant par rapport au poids total du comprimé, ledit agent gélifiant étant choisi parmi les gommes d'origine végétale de nature glucidique, les polysaccharides linéaires et linéaires substitués, les polyéthylène glycol, la méthylcellulose et l'hydroxypropylcellulose ;
de 3 à 15% en poids d'un agent générateur de gaz par rapport au poids total du comprimé ; et
de 1 à 30% en poids d'un superdésintégrant par rapport au poids total du comprimé ;
ledit comprimé comprenant moins de 5% d'alginate de sodium en poids par rapport au poids total du comprimé.

2. Comprimé selon la revendication 1, comprenant des granules de Baclofène, de mannitol et éventuellement d'un liant au sein d'une matrice comprenant au moins un agent gélifiant, un agent générateur de gaz et un superdésintégrant.

3. Comprimé selon la revendication 1 ou 2, comprenant 5% en poids de générateur de gaz par rapport au poids total du comprimé.

4. Comprimé selon l'une quelconque des revendications précédentes, ne comprenant pas d'alginate de sodium.

5. Comprimé selon l'une quelconque des revendications précédentes, dans lequel l'agent gélifiant et le générateur de gaz se présentent dans un rapport en poids agent gélifiant: générateur de gaz de 1:10 à 10:1.

6. Comprimé selon l'une quelconque des revendications précédentes, pour son utilisation en tant que médicament.

7. Comprimé selon l'une quelconque des revendications 1 à 5, pour son utilisation dans le traitement de la dépendance à l'alcool ou du maintien du sevrage des alcooliques.

## Patentansprüche

1. Pharmazeutische Matrixtablette, die auf oralem Weg ein bis zwei Mal pro Tag verabreichbar ist, mit kontrollierter Freisetzung von Baclofen durch Retention im Magen, umfassend Granulate, die durch feuchte Granulierung von Baclofen erhalten werden, von einem oder mehreren Verdünnern, ausgewählt aus den Cellulosederivaten wie der mikrokristallinen Zellulose und den Polyolen wie Mannitol, und gegebenenfalls von einem Bindemittel innerhalb einer Matrix, umfassend:
5 bis 50 Gew.-% von mindestens einem Geliermittel im Verhältnis zum Gesamtgewicht der Tablette, wobei das Geliermittel aus den Gummis pflanzlichen Ursprungs des Glucidtyps, den linearen und substituierten linearen Polysacchariden, den Polyethylenglucolen, der Methylcellulose und der Hydroxypropylcellulose ausgewählt ist;
3 bis 15 Gew.-% eines Gaserzeugungsmittels im Verhältnis zum Gesamtgewicht der Tablette; und
1 bis 30 Gew.-% eines Superzerfallsmittels im Verhältnis zum Gesamtgewicht der Tablette;
wobei die Tablette weniger als 5 Gew.-% Natriumalginat im Verhältnis zum Gesamtgewicht der Tablette umfasst.

2. Tablette nach Anspruch 1, umfassend Granulate von Baclofen, Mannitol und gegebenenfalls von einem Bindemittel innerhalb einer Matrix, die mindestens ein Geliermittel, ein Gaserzeugungsmittel und ein Superzerfallsmittel umfasst.

3. Tablette nach Anspruch 1 oder 2, umfassend 5 Gew.-% Gaserzeuger im Verhältnis zum Gesamtgewicht der Tablette.

4. Tablette nach einem der vorangehenden Ansprüche, die kein Natriumalginat umfasst.

5. Tablette nach einem der vorangehenden Ansprüche, wobei das Geliermittel und der Gaserzeuger in einem Gewichtsverhältnis Geliermittel : Gaserzeuger von 1 : 10 bis 10 : 1 vorliegen.

6. Tablette nach einem der vorangehenden Ansprüche für ihre Verwendung als Arzneimittel.

7. Tablette nach einem der Ansprüche 1 bis 5 für ihre Verwendung bei der Behandlung der Alkoholabhängigkeit oder der Aufrechterhaltung der Abstinenz von Alkoholabhängigen.

## Claims

1. Matrix-type pharmaceutical tablet administrable by oral route once to twice per day with controlled release of baclofen by gastric retention comprising granules obtained by wet granulation of Baclofen, of one or more diluents selected from cellulose derivatives such as microcrystalline cellulose and polyols such as mannitol, and optionally a binder within a matrix comprising:
from 5 to 50% by weight of gelling agent relative to the total weight of the tablet, said gelling agent being selected from gums of plant origin of carbohydrate nature, linear and substituted linear polysaccharides, polyethylene glycol, methylcellulose and hydroxypropylcellulose;
from 3 to 15% by weight of a gas-generating agent relative to the total weight of the tablet; and
from 1 to 30% by weight of a superdisintegrant relative to the total weight of the tablet;
said tablet comprising less than 5% by weight of the total weight of the tablet of sodium alginate.

2. Tablet according to claim 1, comprising baclofen granules, mannitol and optionally a binder within a matrix comprising at least a gelling agent, a gas-generating agent and a superdisintegrant.

3. Tablet according to claim 1 or 2, comprising 5% by weight of a gas-generating agent relative to the total weight of the tablet.

4. Tablet according to any one of the preceding claims, not comprising sodium alginate.

5. Tablet according to any one of the preceding claims, wherein the gelling agent and the gas generator are in a weight ratio of gelling agent to gas generator of 1:10 to 10:1.

6. Tablet according to any one of the preceding claims, for use as a drug.

7. Tablet according to any one of claims 1 to 5, for use in the treatment of alcohol dependence or the maintaining of alcohol abstinence.
